# EUROPÄISCHE PATENTANMELDUNG

(11) **EP 3 090 767 A1**
(43) Veröffentlichungstag der Anmeldung: **09.11.2016**
(21) Anmeldenummer: 15166554.4
(22) Anmeldetag: 06.05.2015
(51) Int. Cl.: A61M 1/12

(54) **HERZPUMPENEINRICHTUNG UND HERZPUMPENEINRICHTUNGSSYSTEM**

(71) Anmelder: Berlin Heart GmbH, 12247 Berlin (DE)
(72) Erfinder: Winterwerber, Kim Peter, Dr., 12527 Berlin (DE); Krambeck, Helge, 12163 Berlin (DE); Kallenbach, Sebastian, 13353 Berlin (DE); Strommenger, Daniel, 12161 Berlin (DE)
(74) Vertreter: Pfenning, Meinig & Partner mbB

(57) **Zusammenfassung**

Die Erfindung bezieht sich auf eine Herzpumpeneinrichtung mit einer Herzpumpe (3) sowie einem mit dieser verbundenen Steuergerät (5,5'), das ein erstes Verbinderelement (8a) einer ersten Verbindungseinrichtung (8) aufweist, sowie mit einem Zwischensteckmodul (7), das ein zweites Verbinderelement (8b) aufweist, das mit dem ersten Verbinderelement (8a) lösbar verbindbar ist, wobei das Zwischensteckmodul (7) eine unlösbare oder im Rahmen einer zweiten Verbindungseinrichtung lösbare zweite Verbindungseinrichtung (13, 13a, 14, 15) zur Verbindung mit einer elektrischen Energiequelle (9, 10, 11), insbesondere einem Netzteil oder einem Akku, und zudem eine unlösbare oder im Rahmen einer dritten Verbindungseinrichtung (16a) lösbare dritte Verbindungseinrichtung (16) zur Verbindung mit einer Datenverarbeitungseinrichtung (12), insbesondere einem Terminal, aufweist.

Durch die sekundärseitigen Verbindungsmöglichkeiten des Zwischensteckmoduls (7) werden Verbindungsfehler zu angeschlossenen Akkus, Netzteilen oder anderen anzuschließenden Geräten minimiert.

## Beschreibung

Die Erfindung liegt auf dem Gebiet der Elektrotechnik und ist mit besonderem Vorteil auf dem Gebiet der Medizintechnik einsetzbar. Konkret befasst sich die Erfindung mit Herzpumpeneinrichtungen und entsprechenden Steuer- und Peripheriegeräten.

Seit einiger Zeit sind zur Unterstützung und teilweise zum Ersatz der Herzfunktion von Patienten implantierbare Herzpumpen bekannt, die beispielsweise im Rahmen von VAD-Systemen (ventricular assist devices) betrieben werden. Derartige implantierbare Pumpen werden üblicherweise durch außerhalb des Patientenkörpers liegende Steuereinheiten bzw. Steuergeräte betrieben, die mittels perkutaner Kabel mit der eigentlichen implantierten Pumpe verbunden sind.

Es sind zum Betrieb derartiger Pumpen üblicherweise elektrische Energiequellen wie Akkumulatoren (Akkus) bzw. Batterien, jedoch auch entsprechende Netzteile, vorgesehen, die mit dem externen Steuergerät der Pumpe verbunden werden. Solche elektrische Energiequellen sind austauschbar, um beispielsweise Akkus wechseln zu können oder den Betrieb an die augenblicklich zur Verfügung stehenden Energieversorgungssysteme anpassen zu können. Das externe Steuergerät tauscht mit den implantierten Elementen, also beispielsweise mit der Herzpumpe selbst, Informationen aus. Diese beziehen sich beispielsweise auf Parameter zur Einstellung des Betriebes der Pumpe, Messdaten über das Betriebsverhalten der Pumpe oder Daten des Patienten. Üblicherweise wird zur Einstellung von Betriebsparametern der Pumpe oder zur Auswertung von Betriebsdaten durch den betreuenden Arzt eine Datenverbindung zwischen einem Steuergerät und einem externen Gerät, einer Datenverarbeitungseinrichtung - in diesem Zusammenhang auch Terminal genannt-, hergestellt. Darüber können die Daten aus dem Steuergerät ausgelesen oder auch Daten im Steuergerät hinterlegt werden.

Während der Zeit des Datenaustausches oder des Tausches einer externen Energiequelle bei der Wartung durch den betreuenden Arzt muss die Energieversorgung der Herzpumpeneinrichtung sichergestellt sein. Es sind daher im Fall der Wartung oft mehrere Geräte an das Steuergerät anzuschließen, womit sich das Risiko für Bedienungs- und Gebrauchsfehler und den Anschluss von falschen oder defekten Komponenten vergrößert. Insbesondere geht eine große Gefahr von einer Trennung des Systems von der Energieversorgung aus. Es ist zwar eine Redundanz der Energieversorgung grundsätzlich gegeben, jedoch wächst mit der Abtrennung von einem Energieversorgungsquelle und dem Rückfall auf eine Reserveebene die Gefahr eines Gesamtausfalls.

Der vorliegenden Erfindung liegt vor dem Hintergrund des Standes der Technik somit die Aufgabe zugrunde, eine Herzpumpeneinrichtung zu schaffen, bei der Bedienungsfehler minimiert sind und ein Ausfall von Teilen des Systems möglichst geringe Folgen hat.

Die Aufgabe wird mit den Merkmalen der Erfindung gemäß Patentanspruch 1 gelöst. Die Unteransprüche 2 bis 9 stellen vorteilhafte Ausgestaltungen der Erfindung dar. Der Patentanspruch 12 bezieht sich auf ein erfindungsgemäßes Herzpumpeneinrichtungssystem.

Demgemäß wird die Aufgabe gemäß der vorliegenden Erfindung gelöst durch eine Herzpumpeneinrichtung mit einer Herzpumpe sowie einem mit dieser verbundenen Steuergerät, das ein erstes Verbinderelement einer ersten Verbindungseinrichtung aufweist, sowie mit einem Zwischensteckmodul, das ein zweites Verbindereiement aufweist, das mit dem Steuergerät (in manchen Varianten mit dem ersten Verbinderelement) lösbar verbindbar ist, wobei das Zwischensteckmodul eine unlösbare oder im Rahmen einer zweiten Verbindungseinrichtung lösbare zweite Verbindungseinrichtung zur Verbindung mit einer elektrischen Energiequelle, insbesondere einem Netzteil oder einem Akku, und/oder zudem eine unlösbare oder im Rahmen einer dritten Verbindungseinrichtung lösbare dritte Verbindungseinrichtung zur Verbindung mit einer Datenverarbeitungseinrichtung, insbesondere einem Terminal, aufweist.

Oft ist eine derartige Herzpumpe implantiert und mittels einer perkutanen Leitungsverbindung mit einem Steuergerät verbunden. Die Pumpe selbst kann dabei jedoch auch außerhalb des Patientenkörpers angeordnet und mittels Kathetern an Elemente des Blutkreislaufsystems zur Entnahme und Einspeisung von Blut angeschlossen sein. Üblicherweise kann im Rahmen dieser Verbindung zwischen der Herzpumpe und dem Steuergerät eine Verbindungseinrichtung vorgesehen sein.

Zudem ist eine erste Verbindungseinrichtung vorgesehen, mittels deren das Zwischensteckmodul an das Steuergerät angesteckt werden kann. Das Zwischensteckmodul seinerseits weist eine zweite Verbindungseinrichtung zur Verbindung mit einer elektrischen Energiequelle sowie eine dritte Verbindungseinrichtung zur Verbindung mit einer Datenverarbeitungseinrichtung, insbesondere einem Terminal, auf. Damit wird das Zwischensteckmodul nur mit einem einzigen Verbinderelement mit dem Steuergerät verbunden und bietet seinerseits zwei Verbindungseinrichtungen für eine elektrische Energiequelle und eine Datenverarbeitungseinrichtung, wobei jede von ihnen einzeln oder beide als Verbindungseinrichtungen ausgebildet sein können. Es können entweder das Energieversorgungsmodul oder die Datenverarbeitungseinrichtung oder beide unlösbar mit dem Zwischensteckmodul verbunden sein, oder für eines oder beide dieser externen Elemente steht jeweils eine gesonderte Verbindungseinrichtung am Zwischensteckmodul zur Verfügung. Entsprechende Verbindungseinrichtungen können derart ausgebildet sein, dass die Verwechslung beispielsweise eines Steckplatzes nicht möglich ist, so dass jedes Verbinderelement, beispielsweise einer elektrischen Energiequelle oder einer Datenverarbeitungseinrichtung, jeweils nur an ein einziges Verbinderelement an dem Zwischensteckmodul ansteckbar ist. Verwechslungen und Fehlverbindungen sind damit ausgeschlossen.

Üblicherweise gibt es im Stand der Technik jeweils bei einem Steuergerät eine konventionelle Verbindungseinrichtung, die ein Verbinderelement aufweist, das mit einem einzigen Verbinderelement von Peripheriegeräten verbindbar ist. Dabei war in der Vergangenheit bei der Belegung der Steckverbindung mit der Verbindung zu einer Datenverarbeitungseinrichtung die Gefahr verbunden, die Herzpumpeneinrichtung vollständig von einer externen Energieversorgung zu trennen. Es konnte beim Stand der Technik auch passieren, dass eine elektrische Energiequelle irrtümlich an eine Datenverbindung angesteckt wurde.

Durch die Verzweigung der unterschiedlichen Zwecken dienenden Anschlüsse innerhalb des Zwischensteckmoduls auf zwei gesonderte Verbindungen werden Verwechslungen ausgeschlossen. Durch entsprechend unterschiedliche Formgebung der Verbindungseinrichtungen können Fehlverbindungen weiter vermieden werden.

Das Verbinderelement kann dabei beispielsweise ein Steckverbindungselement, ein optisches Verbindungselement, und/oder ein drahtloses, funkbasiertes Verbindungselement sein. Das Verbinderelement kann also sowohl als physisches Verbinderelement als auch als virtuelles Verbinderelement zur Übertragung von Daten aus dem Steuergerät an ein weiteres mit dem Zwischensteckmodul verbundenes Interface, wie einen externen Programmierer oder ein externes Auslese- und/oder Wartungsmodul, angeschlossen werden. In einer weiteren Ausführungsform ist die Verbindungseinrichtung für eine induktive Kopplung ausgelegt. Das entsprechende Verbinderelement kann beispielsweise als Spule ausgebildet sein, welche derart angeordnet ist, dass eine entsprechende Spule zur maximalen induktiven Kopplung ausgerichtet werden kann. Hierbei können die beiden Spulen beispielsweise konzentrisch zueinander ausgerichtet werden.

Eine vorteilhafte Ausgestaltung der Erfindung sieht vor, dass das Steuergerät mittels einer nicht drahtgebundenen Verbindung, insbesondere einer Funkverbindung, mit der Datenverarbeitungseinrichtung, insbesondere dem Terminal, verbunden ist. Damit wird eine mehrkanalige Verbindung zwischen dem Steuergerät und der Datenverarbeitungseinrichtung oder einem Terminal hergestellt, die für eine erhöhte Verfügbarkeit durch Redundanz sorgt. Die nicht drahtgebundene Verbindung kann dabei mittels gängiger WLAN- bzw. Bluetooth-Standards oder auch nach anderen Kommunikationsstandards funktionieren und die primär genutzte Verbindung oder auch die sekundär genutzte Verbindung sein.

Eine weitere vorteilhafte Ausgestaltung der Erfindung sieht, wie bereits oben angedeutet, vor, dass das Zwischensteckmodul je ein gesondertes Verbinderelement der zweiten und der dritten Verbindungseinrichtung aufweist. Auf diese Weise können die Steckverbinder der zweiten und dritten Steckverbindung geometrisch unterschiedlich ausgestaltet werden, so dass eine Verwechslung oder auch überhaupt ein Einstecken des jeweils falschen Steckverbinders in oder an einen falschen an dem Zwischensteckmodul vorhandenen Steckverbinder unmöglich gemacht ist.

Eine weitere vorteilhafte Ausgestaltung der Erfindung kann vorsehen, dass das Zwischensteckmodul mittels einer zweiten Verbindungseinrichtung mit einer elektrischen Energiequelle verbunden ist, wobei von der Leitung zwischen dem Zwischensteckmodul und dem Energieversorgungsaggregat ein Verbinderelement der dritten Verbindungseinrichtung abzweigt. In diesem Fall ist dem Nutzer des Zwischensteckmoduls nahegelegt, zunächst die zweite Verbindungseinrichtung zu verbinden und damit das Zwischensteckmodul mit einer elektrischen Energiequelle zu verbinden und erst dann die dritte Steckverbindung herzustellen, indem ein entsprechender Steckverbinder an das Verbinderelement angesteckt wird, das an der Leitung zwischen dem Zwischensteckmodul und der elektrischen Energiequelle vorgesehen ist. Hierdurch ist in hohem Maße sichergestellt, dass bei Herstellung der Verbindung zwischen dem Steuergerät und einer Datenverarbeitungseinrichtung / einem Terminal gesichert ist, dass das Steuergerät bereits dauerhaft mit einem Energieversorgungsaggregat verbunden ist.

Eine weitere vorteilhafte Ausgestaltung der Erfindung sieht vor, dass das Steuergerät eine Ausnehmung aufweist, in die das Zwischensteckmodul einschiebbar ist und in der ein erstes Verbindereiement der ersten Verbindungseinrichtung angeordnet ist. Durch die Form der Ausnehmung kann sichergestellt werden, dass nur entsprechende Zwischensteckmodule, die eine angepasste Form haben, an das Verbinderelement der ersten Verbindungseinrichtung angesteckt werden können. Die Form der Ausnehmung kann beispielsweise auch an die Form eines an dieselbe Verbindungseinrichtung ansteckbaren Akkus als elektrischer Energiequelle angepasst sein.

Zudem kann vorteilhaft vorgesehen sein, dass wenigstens ein, insbesondere zwei elektrische Steckkontakte des ersten Verbinderelements sowohl für die Verbindung des Steuergeräts zu einer Energieversorgungsquelle als auch für die Verbindung zu einer Datenverarbeitungseinrichtung vorgesehen sind.

Üblicherweise sind bei Verbindung des Steuergeräts mit einer elektrischen Energiequelle einzelne Pins einer Leitungsverbindung bereits sowohl für die Energieübertragung als auch für eine Datenübertragung verwendet. Es wird hierzu zur Trennung der Funktionen beispielsweise ein I²C-Bus verwendet, um Informationen von einem Akku auslesen zu können. Umgekehrt können auch akkuseitig die entsprechenden Anschlüsse zur Identifizierung der Kontaktierung/Konnektierung benutzt werden.

Die bei der ersten Verbindungseinrichtung zur Verfügung stehenden Verbindungsleiter können gleichzeitig zur Übermittlung von Daten von und zu einer elektrischen Energiequelle auch zur Verbindung des Steuergeräts mit einer Datenverarbeitungseinrichtung / einem Terminal und dort ebenfalls zum Austausch von Daten verwendet werden. Zudem können die gleichen oder zumindest einige Pins der Verbindungseinrichtung zur Übertragung elektrischer Energie verwendet werden.

Dabei kann vorteilhaft vorgesehen sein, dass das Zwischensteckmodul mittels einer Datenbusverbindung mit dem Steuergerät verbindbar ist.

Um die vielfältigen Verbindungsfunktionen der ersten Verbindungseinrichtung bewältigen zu können, kann vorteilhaft vorgesehen sein, dass das Zwischensteckmodul eine Multiplexeinrichtung, insbesondere eine Zeitmultiplexeinrichtung, zur Verbindung der ersten Verbindungseinrichtung wechselweise mit der zweiten und dritten Verbindungseinrichtung aufweist. Damit kann die Multiplexeinrichtung periodisch zwischen einer Verbindung der Energieversorgungsquelle über den Zwischenstecker mit dem Steuergerät einerseits und einer Verbindung einer Datenverarbeitungseinrichtung / eines Terminals über das Zwischensteckmodul mit dem Steuergerät andererseits umschalten. Diese beiden Verbindungen können damit entkoppelt werden.

In einer weiteren Ausgestaltung ist das Steuergerät fest mit der verbundenen Energiequelle, insbesondere einer Batterie, versehen.

Die Erfindung bezieht sich außer auf eine Herzpumpeneinrichtung der oben beschriebenen Art zudem auch auf ein Herzpumpeneinrichtungssystem mit einer Herzpumpeneinrichtung nach einem der Ansprüche 1 bis 11 sowie mit einer Energiespeichereinrichtung, die anstelle des Zwischensteckmoduls mit dem ersten Verbinderelement lösbar verbindbar ist. In für einen Patienten vorteilhafter Weise kann damit in den meisten Fällen das Steuergerät mit einer elektrischen Energiequelle verbunden sein; beispielsweise kann in einer Ausnehmung an dem Steuergerät ein Akku eingesteckt werden, während zu bestimmten Zeiten, beispielsweise zur Wartung, ein Zwischensteckmodul anstelle der Energiespeichereinrichtung eingesteckt wird. Für den Patienten kann das Herzpumpeneinrichtungssystem mit dem Zwischensteckmodul und der Energiespeichereinrichtung als Set zur Verfügung stehen.

In einem weiteren Aspekt behandelt die vorliegende Anmeldung ein Steuergerät für eine Herzpumpeneinrichtung. Das Steuergerät umfasst dabei eine Steuereinrichtung zur Steuerung einer Herzpumpe sowie ein Gehäuse mit einer Verbindungseinrichtung, welche genau ein Verbinderelement, beispielsweise ein Steckverbinderelement oder ein anderes der vorab beschriebenen Verbinderelement, zum lösbaren Verbinden des Steuergeräts mit einem Zwischensteckmodul besitzt. Ein derartiges Steuergerät umfasst neben einer eventuellen Verbindungseinrichtung zur Herzpumpe selbst lediglich genau ein Verbinderelement für ein Zwischensteckmodul. Das Verbinderelement kann dabei beispielsweise ein Steckverbindungselement, ein optisches Verbindungselement und/oder ein drahtloses, funkbasiertes Verbindungselement sein. Das Verbinderelement kann also sowohl als physisches Verbinderelement als auch als rein virtuelles Verbinderelement zur Übertragung von Daten aus dem Steuergerät an ein weiteres mit dem Zwischensteckmodul verbundenes Interface, wie einen externen Programmierer oder ein externes Auslese- und/oder Wartungsmodul, angeschlossen werden.

Das Steuergerät weist also lediglich ein einziges Verbinderelement auf. Hierdurch kann auf weitere Verbinderelemente verzichtet werden, was die Wahrscheinlichkeit einer Fehlbedienung oder Fehlverbindung des Steuergeräts mit einem Zwischensteckmodul drastisch reduziert. Das genau eine Verbinderelement ist dabei innerhalb des Gehäuses oder an einer Außenseite des Gehäuses angeordnet.

In einer weiteren Ausführungsform ist innerhalb des Gehäuses eine fest mit dem Steuergerät verbundene Energiequelle, insbesondere eine Batterie, angeordnet. Diese "interne" Energiequelle dient beispielsweise einer Überbrückung einer externen Energieversorgung, welche beispielsweise ausgetauscht werden soll. Zudem wird eine Energieversorgung während eines Ausfalls des Verbinderelements oder der externen Energiequelle vermieden.

In einer weiteren Ausführungsform umfasst die Verbindungseinrichtung des Steuergeräts eine mit dem Verbinderelement verbundene Schalteinheit, welche derart konfiguriert ist, dass Steuersignale sowohl einer externen Energiequelle als auch Steuersignale eines externen Benutzerterminals durch die Steuereinrichtung verarbeitbar sind. Hierdurch wird beispielsweise gewährleistet, dass sowohl reine Energiequellen (ohne weiteren Datenaustausch), welche über das genau eine Verbinderelement mit dem Steuergerät verbunden werden, als auch reine Datenauswertungseinheiten, welche lediglich Daten, jedoch keine zum Betrieb der Herzpumpe notwendige Energie zur Verfügung stellen, mit dem Verbinderelement gekoppelt werden können. Die Schalteinheit kann beispielsweise eine Kommunikationsschnittstelle umfassen, welche einen im Steuergerät angeordneten Mikrocontroller, einen Prozessor oder ein Field Programmable Array abwechselnd mit den Steuersignalen einer externen Energiequelle, wie beispielsweise einen Akkumulator, und einer externen Datenverarbeitungseinrichtung verbindet. Die Schalteinheit kann dabei in manchen Ausführungsbeispielen feststellen, ob die am Verbinderelement auftreffenden Steuersignale von einer Energiequelle und/oder einer weiteren Datenverarbeitungsvorrichtung stammen bzw. ob Steuersignale des Steuergeräts für eine externe Energiequelle oder eine weitere Datenverarbeitungsvorrichtung bestimmt sind. Alternativ kann ein Übertragungsprotokoll gewählt werden, welches in festen zeitlichen Abständen zwischen einer eventuell vorhandenen Energiequelle und einer eventuell vorhandenen Datenverarbeitungsvorrichtung schaltet.

Im Folgenden wird die Erfindung anhand von Ausführungsbeispielen in Figuren einer Zeichnung gezeigt und anschließend beschrieben. Dabei zeigt
- Fig. 1: eine schematische Ansicht eines Patientenkörpers im Ausschnitt mit einer implantierten Herzpumpe sowie einem Steuergerät,
- Fig. 2: schematisch ein Steuergerät mit einem Zwischensteckmodul,
- Fig. 3: drei verschiedene Kombinationen von jeweils einem Zwischensteckmodul mit einem oder mehreren mit diesem verbundenen Energiequellen,
- Fig. 4: schematisch einen Multiplexer,
- Fig. 5: den detaillierteren Aufbau eines Steuergeräts; sowie
- Fig. 6: eine Variante einer Steuereinrichtung eines Steuergeräts.

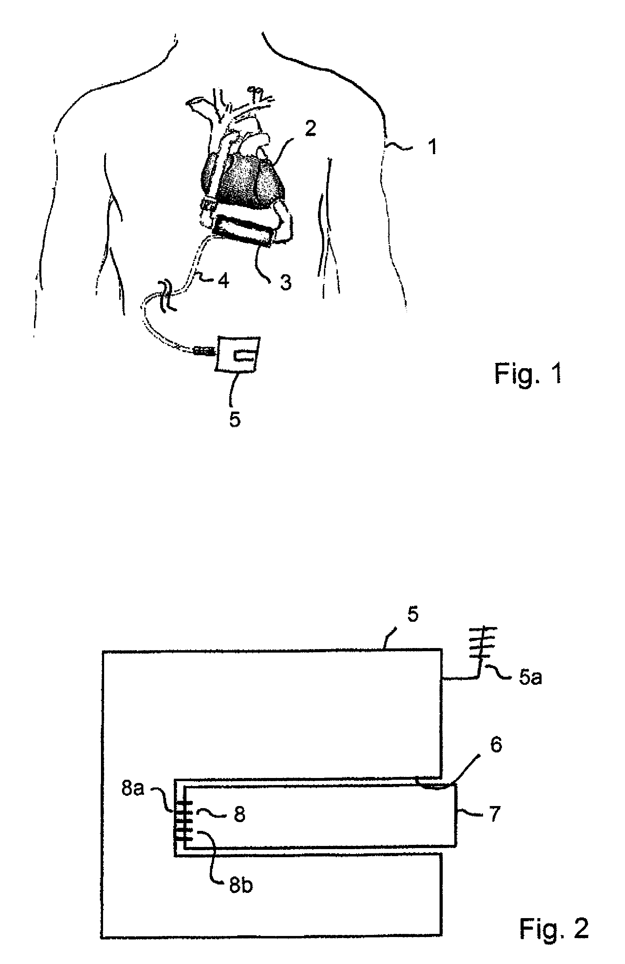

Figur 1 zeigt eine Ansicht eines Patientenkörpers 1 mit einem Patientenherzen 2, an das als VAD (ventricular assist device) eine Herzpumpe 3 angeschlossen ist. Die Herzpumpe 3 ist in dem Patientenkörper implantiert und mittels einer Verbindungsleitung 4 transkutan mit einem Steuergerät 5 außerhalb des Patientenkörpers verbunden. Über das Steuergerät 5 werden sowohl Daten, wie beispielsweise Pumpenparameter, als auch elektrische Energie mit der Herzpumpe 3 ausgetauscht.

In Figur 2 ist vergrößert ein Steuergerät schematisch dargestellt, wobei in eine Ausnehmung 6 des Steuergeräts 5 ein Zwischensteckmodul 7 eingesetzt ist. Das Zwischensteckmodul 7 ist mittels einer ersten Verbindungseinrichtung 8 mit dem Steuergerät 5 verbunden. Die erste Verbindungseinrichtung 8 sieht ein erstes Verbinderelement 8a auf der Seite des Steuergeräts 5 und ein zweites Verbinderelement 8b auf der Seite des Zwischensteckmoduls 7 vor. Dabei kann das erste Verbinderelement 8a ein Stecker und das zweite Verbinderelement 8b eine Buchse sein oder umgekehrt, oder die Verbinderelemente 8a und 8b können jeweils einen Stecker oder eine Buchse umfassen, oder es kann auch eine Anzahl gemischter Kontakte auf beiden Seiten der ersten Verbindungseinrichtung vorgesehen sein, wobei auf beiden Seiten sowohl Steckerelemente als auch Buchsen vorgesehen sind. Wichtig ist in diesem Zusammenhang, dass die erste Verbindungseinrichtung 8 problemlos steckbar und lösbar ist. Die eigentlichen Verbinderelemente 8a, 8b liegen dabei geschützt in der Ausnehmung 6 des Steuergeräts 5.

Die Erfindung ist grundsätzlich mit größtem Vorteil bei implantierten Herzpumpen einsetzbar, jedoch ist ein Einsatz auch bei nichtimplantierten, außerhalb des Patientenkörpers angeordneten und über Kanülen mit dem Kreislauf eines Patienten verbundenen künstlichen Herzen einsetzbar.

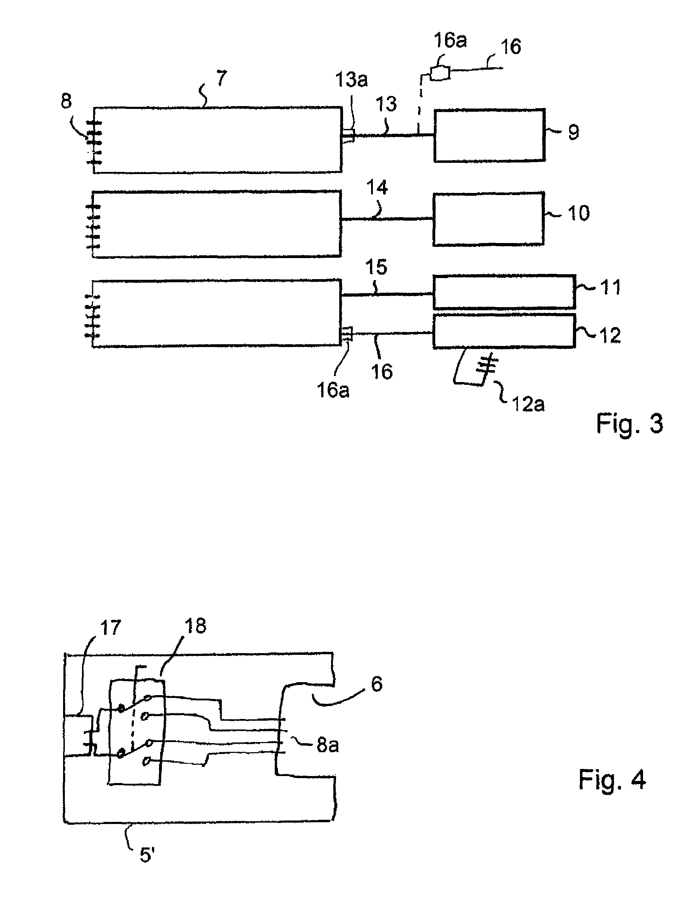

In Figur 3 ist dargestellt, dass das Zwischensteckmodul sekundärseitig, d. h. bezüglich der dem Steuergerät 5 und der ersten Verbindungseinrichtung 8 abgewandten Seite, mit verschiedenen weiteren Elementen und/oder Energiequellen verbunden sein kann. In Figur 3 ist das Zwischensteckmodul 7 in drei Varianten untereinander dargestellt, wobei in der obersten Variante das Zwischensteckmodul mit einem Netzgerät 9 verbunden ist, während in der mittleren Darstellungsvariante das Zwischensteckmodul mit einem Energieversorgungsquelle in Form eines Akkus 10 verbunden ist. In der am weitesten unten dargestellten Variante ist das Zwischensteckmodul sowohl mit einem Energieversorgungselement 11, entweder in Form eines Akkus oder in Form eines Netzteils, als auch mit einer Datenverarbeitungseinrichtung 12 in Form eines Terminals verbunden. Es sind jeweils zur Verbindung der einzelnen Energiequellen 9, 10, 11, 12 Leitungen 13, 14, 15, 16 dargestellt, die die einzelnen Elemente mit dem Zwischensteckmodul 7 verbinden. Dabei kann grundsätzlich jede der Leitungen 13,14,15,16 mittels einer Verbindungseinrichtung 13a, 16a mit dem Zwischensteckmodul 7 verbunden sein. Die Verbindungseinrichtung kann beispielsweise jeweils als Steckverbindungseinrichtung ausgebildet sein. Eine weitere Variante kann jedoch auch sein, dass die Leitung 15 das Zwischensteckmodul mit der Energieversorgungsquelle 11 fest und unlösbar verbindet, während die Datenverarbeitungseinrichtung / das Terminal 12 mit dem Zwischensteckmodul mittels einer steckbaren Leitung 16 im Rahmen einer dritten Verbindungseinrichtung verbindbar ist. Durch diese Konstellation ist eine dauerhafte Verbindung des Steuergeräts mit einer Energieversorgung sichergestellt.

Dabei kann es vorteilhaft weiter vorgesehen sein, dass für den Fall, dass sowohl die Leitung 15 als auch die Leitung 16 mittels einer Verbindungseinrichtung mit dem Zwischensteckmodul verbindbar sind, die entsprechenden Verbinderelemente derart unterschiedlich ausgebildet sind, dass eine Verwechslung beim Einstecken unmöglich gemacht wird.

Die Datenverarbeitungseinrichtung 12 kann auch mit einer Sende-/Empfangseinrichtung 12a für eine Funkverbindung ausgestattet sein, um mit dem Steuergerät parallel zu der leitungsgebundenen Kommunikation zusätzlich über eine Funkverbindung gekoppelt zu sein.

Figur 4 zeigt schematisch eine Darstellung eines Steuergeräts 5' für eine erfindungsgemäße Herzpumpeneinrichtung mit einer Ausnehmung 6, in der ein Verbinderelement 8a angeordnet ist.

Im Inneren des Steuergeräts 5' ist das sogenannte System symbolisch dargestellt und mit dem Bezugszeichen 17 bezeichnet. in dem System sind alle Funktionselemente des Steuergeräts, die zur Ansteuerung der Herzpumpe 3 dienen, sowie Speichereinrichtungen für Messwerte und Pumpenparameter zusammengefasst. Zwischen dem System 17 und dem Verbinderelement 8a ist eine Multiplexeinrichtung 18 eingezeichnet, die verschiedene Anschlüsse des Verbinderelements nach einem Zeitmultiplexregime mit unterschiedlichen Kommunikationsprotokollen abwechselnd bedient. Dadurch können an die verschiedenen Pins/Kontakte der ersten Verbindungseinrichtung 8 unterschiedliche Geräte angeschlossen werden, die mittels verschiedener Bussysteme und Kommunikationsprotokolle kommunizieren. Innerhalb des Zwischensteckmoduls 7 kann somit die Zahl der Leitungen, die mit dem Verbinderelement 8b verbunden sind, in verschiedene Gruppen aufgeteilt werden, die sekundärseitig an dem Zwischensteckmodul 7 zu unterschiedlichen Leitungen und/oder Verbinderelementen führen. Dort können dann an die verschiedenen Verbinderelemente/Leitungen verschiedene Geräte, wie Netztelle, Akkus/Batteriepacks oder eine Datenverarbeitungseinrichtung, beispielsweise in Form eines Terminals, angeschlossen werden.

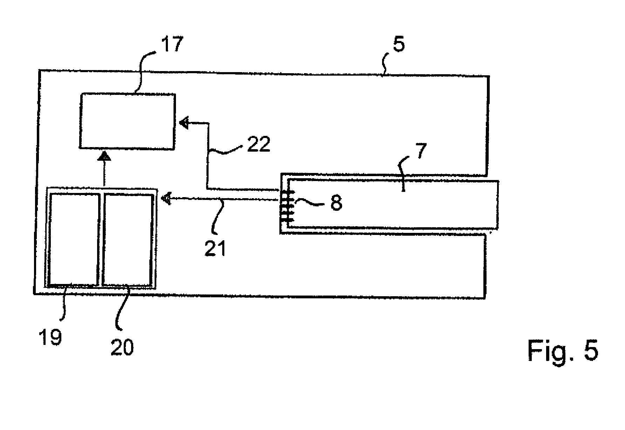

In Figur 5 ist ein Steuergerät 5 mit einem Systemteil 17 schematisch dargestellt, in dem die Funktionselemente des Steuergeräts, wie Prozesssteuerung für die Pumpe, Speichereinrichtungen für Pumpenparameter und dergleichen sowie die Überwachung der Energieversorgung, des Ladezustands der Batterien usw., untergebracht sind. Mit dem System 17 ist einerseits die Einheit mit zwei Energieversorgungsquellen 19, 20 verbunden, wobei diese interne Batterien darstellen, die jeweils das System und die Herzpumpe für etwa fünfzehn Minuten mit Energie versorgen können, beispielsweise während eine externe Batterie gewechselt oder ein externes Netzteil angeschlossen oder ausgewechselt wird.

Andererseits ist das System 17 mit der ersten Verbindungseinrichtung 8 und hierüber mit dem Zwischensteckmodul 7 verbunden. Wird über das Zwischensteckmodul 7 die Verbindung zu einem externen Akku oder einem Netzgerät hergestellt, so kann über die Ladeleitung 21 jede der beiden internen Batterien 19, 20 aufgeladen werden. Durch eine externe Energieversorgungsquelle können zudem über die weitere Leitung 22 das System 17 und die an diese unmittelbar angeschlossene Herzpumpe mit Energie versorgt werden.

Grundsätzlich sind die Verbindungsleitungen 13, 14, 15, 16 zumindest optional als Datenbusverbindungen ausgebildet, d. h., bei der Verbindung 16 zwischen dem Zwischensteckmodul 7 und einer Datenverarbeitungseinrichtung kann es sich um einen reinen Datenbus handeln, während die übrigen Leitungen 13, 14, 15 zumindest auch die Durchleitung einer Speisespannung bzw. eines Speisestroms als Energieversorgung parallel zu einem Datenverkehrsprotokoll erlauben sollten.

Zudem und parallel zu den oben beschriebenen Verbindungen kann als primäre Verbindung zwischen dem Steuergerät 5 und der Datenverarbeitungseinrichtung 12 eine nicht leitungsgebundene Verbindung, beispielsweise in Form einer Funkverbindung, bestehen, die durch die Antennen 12a in Figur 3 und 5a in Figur 2 angedeutet ist. Die leitungsgebundene Verbindung 16 bildet dann eine Rückfallebene für den Fall, dass die nicht leitungsgebundene Verbindung versagt oder fehlerhaft ist.

Anhand der Figur 5 ist erkennbar, dass das Steuergerät lediglich genau ein Verbinderelement 8 für ein Zwischenmodul umfasst. Weitere Verbinderelemente sind in einer Variante nicht vorgesehen. Das Verbinderelement 8 kann beispielsweise in einer Ausnehmung 25 des Gehäuses (schematisch durch die als Steuergerät 5 markierte Linie angedeutet) angeordnet sein. Ein Zwischenmodul kann ebenfalls ein Gehäuse aufweisen, dessen Außenmaße derart sind, dass diese in der Ausnehmung 25 vorzugsweise formschlüssig aufgenommen werden, so dass das genau eine Verbinderelement 8 des Steuergeräts mit dem Verbinderelement des Zwischenmoduls koppelbar ist. Die Verbinderelemente des Steuergeräts und des Zwischenmoduls sind komplementär zueinander ausgebildet; im vorliegenden Fall ist das Verbinderelement ein Steckverbindungselement. Durch die in der Figur 5 schematisch angedeutete Ausnehmung 25 wird zudem gewährleistet, dass das Gehäuse des Steuergeräts das Zwischenmodul vor zufälligem Herausziehen in einer Richtung 26 schützt, da hier die Innenwände der Ausnehmung das Zwischenmodul vor größeren Bewegungen schützen. Die Ausnehmung kann auch als Port bezeichnet werden, an dessen Wandung ein Verbinderelement zum Verbinden mit einem Verbinderelement des Zwischenmoduls angeordnet ist, bevorzugt am Boden des Ports bzw. der Ausnehmung angeordnet.

In der Figur 6 ist eine Variante einer Steuereinrichtung des Steuergeräts dargestellt. Die Steuereinrichtung 30 umfasst einen Mikrocontroller 31, welcher mit einer Verbindungseinrichtung 32 gekoppelt ist. Die Verbindungseinrichtung umfasst beispielsweise ein Steckverbinderelement 32a, welches mit einem entsprechenden Steckverbinderelement eines Zwischenmoduls koppelbar ist. Da das Steuergerät im vorliegenden Beispiel genau ein Verbinderelement für ein Zwischenmodul umfasst, weist die steuergeräteseitige Ausführung der Verbindungseinrichtung 32 sowohl einen Schaltkreis 33 für Kommunikationssignale bzw. Steuersignale als auch einen Schaltkreis 34 für Energie, d. h. einen Schaltkreis, welcher aus einer am Zwischenmodul angeschlossenen Energiequelle (Netzteil oder Akku) und/oder einer internen Energiequelle des Steuergeräts Energie für die Pumpe zur Verfügung stellt, auf. Der Schaltkreis 33 ist mit dem Mikrocontroller 31 gekoppelt und umfasst neben einem Schaltelement 35 einen Schaltkreis 36 für Steuersignale einer Energiequelle, wie beispielsweise eines Akkus oder eines Netzgerätes, und einen Schaltkreis 37 für Steuersignale eines Benutzerterminals, welches mit dem Steuergerät über das Zwischenmodul koppelbar ist. Das Schaltelement 33 kann dabei abwechselnd den Schaltkreis 36 oder den Schaltkreis 37 mit der Verbindungseinrichtung 32 koppeln, so dass der Mikrocontroller sowohl Steuersignale einer Energiequelle als auch des Benutzerterminals verarbeiten kann. Dabei ist der Mikrocontroller derart ausgebildet, dass beispielsweise unabhängig vom angekoppelten Zwischenmodul Steuersignale des Benutzerterminals oder der Energiequelle verarbeitet werden können bzw. an das Zwischenmodul gesendet werden können. Über den Schaltkreis 34 kann eine nicht eingezeichnete interne Energiequelle des Steuergeräts geladen werden, beispielsweise sofern der Mikrocontroller entsprechende Steuersignale des Schaltkreises 37 empfängt. In anderen Ausführungsbeispielen können andere dem Fachmann bekannte Verfahren zur Steuersignalübertragung gewählt werden.

Im vorliegenden Beispiel kann das Zwischenmodul eine Energiequelle oder ein Benutzerterminal oder beides mit dem Steuergerät verbinden. Auf diese Weise ist die genau eine Verbindungseinrichtung keine Einschränkung der Konnektivität des Steuergeräts. Vielmehr wird das Verbinden des Zwischenmoduls mit dem Steuergerät für den Benutzer stark vereinfacht und die Anwendungssicherheit erhöht. Das Erkennen des Zwischenmoduls und das Verarbeiten der entsprechenden Steuersignale wird durch den Mikrocontroller 31 durchgeführt bzw. gesteuert. Selbstverständlich kann der Mikrocontroller durch beispielsweise einen Prozessor ersetzt oder mit einem Prozessor des Steuergeräts verbunden werden.

Durch die hier beschriebene Erfindung werden die Fehleranfälligkeit und die Gefahr für Bedienungsfehler bei einem Herzpumpensystem beträchtlich verringert. Für den Patienten ergeben sich Kombinationsmöglichkeiten, die seinen Komfort erhöhen.

## Patentansprüche

1. Herzpumpeneinrichtung mit einer Herzpumpe (3) sowie einem mit dieser verbundenen Steuergerät (5), das ein erstes Verbinderelement (8a) einer ersten Verbindungseinrichtung (8) aufweist, sowie mit einem Zwischensteckmodul (7), das ein zweites Verbinderelement (8b) aufweist, das mit dem Steuergerät lösbar verbindbar ist, wobei das Zwischensteckmodul eine unlösbare oder im Rahmen einer zweiten Verbindungseinrichtung lösbare zweite Verbindungseinrichtung (13, 13a, 14, 15) zur Verbindung mit einer elektrischen Energiequelle (9,10, 11), insbesondere einem Netzteil oder einem Akku, und/oder eine unlösbare oder im Rahmen einer dritten Verbindungseinrichtung lösbare dritte Verbindungseinrichtung (16, 16a) zur Verbindung mit einer Datenverarbeitungseinrichtung (12), insbesondere einem Terminal, aufweist.

2. Herzpumpeneinrichtung nach Anspruch 1, **dadurch gekennzeichnet, dass** das Steuergerät (5,5') mittels einer nicht drahtgebundenen Verbindung (5a, 12a), insbesondere einer Funkverbindung, mit der Datenverarbeitungseinrichtung (12), insbesondere dem Terminal, verbunden ist.

3. Herzpumpeneinrichtung nach Anspruch 1 oder 2, **dadurch gekennzeichnet, dass** das Zwischensteckmodul (7) je ein gesondertes Verbinderelement der zweiten und der dritten Verbindungseinrichtung aufweist.

4. Herzpumpeneinrichtung nach Anspruch 1, 2 oder 3, **dadurch gekennzeichnet, dass** das Zwischensteckmodul mittels einer zweiten Verbindungseinrichtung (13a) mit einer elektrischen Energiequelle (9, 10, 11) verbunden ist, wobei mit der Leitung (13, 14, 15) zwischen dem Zwischensteckmodul und dem Energieversorgungsaggregat ein Verbinderelement der dritten Verbindungseinrichtung (16, 16a) verbunden ist.

5. Herzpumpeneinrichtung nach Anspruch 1 oder einem der folgenden, **dadurch gekennzeichnet, dass** das Steuergerät (5, 5') eine Ausnehmung (6) aufweist, in die das Zwischensteckmodul (7) einschiebbar ist und in der ein erstes Verbinderelement (8a) der ersten Verbindungseinrichtung (8) angeordnet ist.

6. Herzpumpeneinrichtung nach Anspruch 1 oder einem der folgenden, **dadurch gekennzeichnet, dass** wenigstens ein, insbesondere zwei elektrische Steckkontakte des ersten Verbinderelements (8a) sowohl für die Verbindung des Steuergeräts (5, 5') zu einer elektrischen Energiequelle (9,10,11) als auch für die Verbindung zu einer Datenverarbeitungseinrichtung (12) vorgesehen sind.

7. Herzpumpeneinrichtung nach Anspruch 1 oder einem der folgenden, **dadurch gekennzeichnet, dass** das Zwischensteckmodul (7) mittels einer Datenbusverbindung mit einer Datenverarbeitungseinrichtung (12) verbindbar ist.

8. Herzpumpeneinrichtung nach Anspruch 1 oder einem der folgenden, **dadurch gekennzeichnet, dass** das Zwischensteckmodul (7) mittels einer Datenbusverbindung mit dem Steuergerät (5, 5') verbindbar ist.

9. Herzpumpeneinrichtung nach Anspruch 1 oder einem der folgenden, **dadurch gekennzeichnet, dass** das Zwischensteckmodul (7) eine Multiplexeinrichtung (18), insbesondere eine Zeitmultiplexeinrichtung, zur Verbindung der ersten Verbindungseinrichtung (8) wechselweise mit der zweiten und dritten Verbindungseinrichtung (13a ,16a) aufweist.

10. Herzpumpeneinrichtung nach Anspruch 1 oder einem der folgenden, **dadurch gekennzeichnet, dass** das Steuergerät (5) mit einer fest mit diesem verbundenen Energiequelle, insbesondere einer Batterie, versehen ist.

11. Herzpumpeneinrichtungssystem mit einer Herzpumpeneinrichtung nach einem der Ansprüche 1 bis 9 sowie mit einer Energiespeichereinrichtung (10,11), die anstelle des Zwischensteckmoduls (7) mit dem ersten Verbinderelement (8a) lösbar verbindbar ist.

12. Steuergerät für eine Herzpumpeneinrichtung, wobei das Steuergerät eine Steuereinrichtung zur Steuerung einer Herzpumpe mittels einer Leitung sowie ein Gehäuse mit einer Verbindungseinrichtung umfasst und die Verbindungseinrichtung genau ein erstes Verbinderelement zum lösbaren Verbinden des Steuergeräts mit einem Zwischensteckmodul besitzt.

13. Steuergerät nach Anspruch 12, wobei im Gehäuse eine fest mit diesem verbundene Energiequelle, insbesondere einer Batterie, angeordnet ist.

14. Steuergerät nach einem der Ansprüche 12 oder 13, wobei die Verbindungseinrichtung eine mit dem ersten Verbinderelement verbundene Schalteinheit umfasst, so dass Steuersignale sowohl einer externen Energiequelle als auch Steuersignale eines externen Benutzerterminals durch die Steuereinrichtung verarbeitbar sind.
